# EUROPEAN PATENT APPLICATION

(11) **EP 4 498 377 A1**
(43) Date of publication of application: **29.01.2025**
(21) Application number: 23895036.4
(22) Date of filing: 22.11.2023
(51) Int. Cl.: G16H 10/20, G16H 10/60, G16H 80/00, G16H 50/20

(54) **METHOD, SERVER, AND PROGRAM FOR MANAGING ELECTRONIC MEDICAL INQUIRY INFORMATION WITH MINIMAL INTERACTION**

(30) Priority: 25.11.2022 KR 20220160163
(71) Applicant: Y-Brain Inc., Seongnam-si, Gyeonggi-do 13449 (KR)
(72) Inventor: LEE, Kiwon, Seongnam-si, Gyeonggi-do 13457 (KR); MAENG, Sang Woo, Yongin-si, Gyeonggi-do 16873 (KR)
(74) Representative: HGF
(86) International application number: PCT/KR2023/018927
(87) International publication number: WO 2024/112109

(57) **Abstract**

The present disclosure relates to a method for managing electronic medical inquiry information with minimal interaction, wherein the method enables a patient to easily input and provide answers to an electronic medical inquiry when visiting any hospital and, by storing personal health records and results of the medical inquiry in a cloud, enables patients and hospitals to easily retrieve information.

## Description

### [TECHNICAL FIELD]

The present disclosure relates to a method for managing electronic medical questionnaire information and more specifically, to a method for managing electronic medical questionnaire information with minimal interaction.

### [BACKGROUND ART]

As the prior art, it has been disclosed that PHR information is transmitted to hospitals through personal consent from a PHR management server, which is not specialized for medical questionnaires, and there were inconveniences in terms of subscription procedures and securing necessary PHR information from the hospital's perspective.

In addition, since the patient's medical questionnaire responses are stored on a hospital server, it is difficult for other hospitals to know about the patient's previous medical questionnaire responses, and since patients cannot check their previous medical questionnaire history, there is a problem in that the patients cannot compare their health conditions with previous health conditions.

Accordingly, there is a need for technology that allows both patients and hospitals to easily conduct electronic medical questionnaires and utilize personal health records and medical questionnaire responses, but such technology is not currently disclosed.

### [DETAILED DESCRIPTION OF THE INVENTION]

### [TECHINICAL PROBLEM]

Embodiments of the present disclosure provide a server for managing electronic medical questionnaire information with minimal interaction.

Further, embodiments of the present disclosure provide a server for managing electronic medical questionnaire information, which allows patients to easily input and provide responses to electronic medical questionnaires regardless of which hospital they visit, and stores personal health records and medical questionnaire responses in a cloud, enabling patients and hospitals to easily look up information.

However, problems to be solved by the present disclosure are may not be limited to the above-described problems. Although not described herein, other problems to be solved by the present disclosure can be clearly understood by those skilled in the art from the following description.

### [TECHNICAL SOLUTION]

According to an embodiment, a method for managing electronic medical questionnaire information with minimal interaction, the method being performed by a server, includes receiving, from a hospital server, a data packet including personal identification information of a patient and a type of a medical questionnaire to be provided to the patient, identifying an account of the patient matching the personal identification information, generating a link including at least one of a personal information collection consent and an electronic medical questionnaire, and providing the generated link to a terminal of the patient, receiving, from the terminal of the patient, medical questionnaire responses for the electronic medical questionnaire, storing the received medical questionnaire responses in a cloud repository corresponding to the account of the patient, and providing the received medical questionnaire response to the hospital server.

The identifying of the account may further include creating a simplified account for the patient based on the personal identification information when the account of the patient does not exist in the server upon identification of the account, and the receiving of the medical questionnaire responses may further include completing subscription for the simplified account when receiving the medical questionnaire responses for the electronic medical questionnaire from the terminal of the patient.

The personal identification information may include a full name and a phone number of the patient.

The server may store the medical questionnaire responses received from the terminal of the patient for the electronic medical questionnaire in a cumulative manner after the subscription is completed, and when a request for provision of the cumulative medical questionnaire responses for the patient is received from a specified hospital server, provide the medical questionnaire responses for a period corresponding to the request for provision of the specified hospital server.

The server may store the medical questionnaire responses in a cumulative manner according to a medical specialty of the patient.

The server may store the medical questionnaire responses received from the terminal of the patient for the electronic medical questionnaire in a cumulative manner after the subscription is completed, when medical questionnaire responses (hereinafter, referred to as 'new medical questionnaire responses') for a new electronic medical questionnaire are received from the terminal of the patient, compare the new medical questionnaire responses with the cumulative medical questionnaire responses to derive differences therebetween, and provide the derived differences to the terminal of the patient.

The server may allow the patient to use the subscribed account identically even when the patient uses a hospital other than the hospital.

The server may be affiliated with a non-medical institution.

According to an embodiment, a server for managing electronic medical questionnaire information with minimal interaction includes a communication device that receives, from a hospital server, a data packet including personal identification information of a patient and a type of a medical questionnaire to be provided to the patient and a processor that identifies an account of the patient matching the personal identification information, generates a link including at least one of a personal information collection consent and an electronic medical questionnaire, the link being to be provided to the patient, provides the generated link to a terminal of the patient, and receives, from the terminal of the patient, medical questionnaire responses for the electronic medical questionnaire, stores the received medical questionnaire responses in a cloud repository corresponding to the account of the patient, and provides the received medical questionnaire response to the hospital server via the communication device.

In addition, a computer program stored in a computer-readable recording medium, which executes a method for implementing the present disclosure may be further provided.

In addition, a computer-readable recording medium for recording a computer program for executing a method for implementing the present disclosure may be further provided.

### [ ADVANTAGEOUS EFFECTS OF THE INVENTION]

According to the aforementioned embodiments of the present disclosure, it is possible to provide the server for managing electronic medical questionnaire information with minimal interaction.

According to the aforementioned embodiments of the present disclosure, a patient may easily input and provide responses to an electronic medical questionnaire regardless of which hospital they visit, and patients and hospitals easily look up information by storing personal health records and medical questionnaire responses in a cloud.

However, effects of the present disclosure may not be limited to the above-described effects. Although not described herein, other effects of the inventive concept may be clearly understood by those skilled in the art from the following description.

### [ DESCRIPTION OF THE DRAWINGS]

FIG. 1 is a diagram illustrating an example of a conventional method of collecting PHR information.
FIG. 2 is a diagram illustrating an example of a method for managing electronic medical questionnaire information with minimal interaction according to an embodiment of the present disclosure.
FIG. 3 is a schematic diagram of a system for managing electronic medical questionnaire information with minimal interaction according to an embodiment of the present disclosure.
FIG. 4 is a block diagram of a server for managing electronic medical questionnaire information with minimal interaction according to an embodiment of the present disclosure.
FIG. 5 is a flowchart of a method for managing electronic medical questionnaire information with minimal interaction according to an embodiment of the present disclosure.
FIG. 6 is a flowchart illustrating a case in FIG. 5 where the patient's account does not exist.
FIG. 7 is a flowchart illustrating a case in FIG. 5 where the patient's account exists.
FIG. 8 illustrates an example in which an account is created/verified for the same server even when the same patient visits various hospitals.
FIG. 9 illustrates an example in which various medical questionnaire responses of the same patient are stored in the same account in the cloud repository according to FIG. 8.
FIG. 10 is a diagram illustrating an example in which an artificial intelligence model compares the patient's medical questionnaire responses at different times to derive differences and providing the differences to the patient.

### [ BEST MODE]

Like reference numerals refer to like elements throughout the present disclosure. The present disclosure does not describe all elements of the embodiments, and general content in the technical field to which the present disclosure pertains or overlapping content between embodiments are omitted. Terms such as "unit", "module", "member", and "block" may be embodied as hardware or software. According to embodiments, a plurality of "unit", "module", "member", and "block" may be implemented as a single component or a single "unit", "module", "member", and "block" may include a plurality of components.

It will be understood that when an element is referred to as being "connected" another element, it can be directly or indirectly connected to the other element, wherein the indirect connection includes "connection via a wireless communication network".

Also, when a part "includes" or "comprises" an element, unless there is a particular description contrary thereto, the part may further include other elements, not excluding the other elements.

Throughout the specification, a certain member being located "on" another member includes not only a case where the certain member is in contact with another member, but also a case where another member exists between two members.

Terms such as first and second are used to distinguish one component from another component, and the components are not limited by the above-mentioned terms.

As used herein, singular forms may include plural forms as well unless the context clearly indicates otherwise.

In each step, identification symbols are used for convenience of description and the identification symbols do not describe the order of the steps and the steps may be performed in a different order from the described order unless the context clearly indicates a specific order.

Hereinafter, the operating principle and embodiments of the present disclosure will be described with reference to the attached drawings.

A system for managing electronic medical questionnaire information with minimal interaction according to embodiments of the present disclosure may be performed by a device for managing electronic medical questionnaire information with minimal interaction, which may provide an electronic medical questionnaire information management service to hospitals, users (patients) affiliated/subscribed to the services via wired/wireless communication, including a server device.

In an embodiment of the present disclosure, a server 100 providing the electronic medical questionnaire information management service may be affiliated with a non-medical organization.

Accordingly, the following embodiments will be described under assumption that the server for managing electronic medical questionnaire information with minimal interaction 100 is the subject of operation.

As used herein, the "server for managing electronic medical questionnaire information with minimal interaction 100 according to the present disclosure" includes all of the various devices capable of performing computational processing and providing results to a user. For example, a device for managing electronic medical questionnaire information with minimal interaction according to the present disclosure may include a computer, the server 100, and a portable terminal, or may be in the form of any one thereof.

Here, the computer may include, for example, a notebook, desktop, laptop, tablet PC, slate PC, or the like, which is equipped with a web browser.

The server device 100 is a server that processes information by communicating with an external device, and may include an application server, a computing server, a database server, a file server, a game server, a mail server, a proxy server, a web server and/or the like.

The portable terminal may be, for example, a wireless communication device that is portable and mobile and may include all types of handheld-type wireless communication devices, such as PCS (Personal Communication System), GSM (Global System for Mobile communications), PDC (Personal Digital Cellular), PHS (Personal Handyphone System), PDA (Personal Digital Assistant), IMT (International Mobile Telecommunication)-2000, CDMA (Code Division Multiple Access)-2000, W-CDMA (W-Code Division Multiple Access), or WiBro (Wireless Broadband Internet) terminals, and wearable devices such as watches, rings, bracelets, anklets, necklaces, glasses, contact lenses, or head-mounted-device (HMD), or the like.

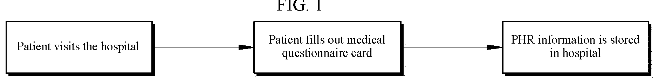

FIG. 1 is a diagram illustrating an example of a conventional method of collecting PHR information.

Before describing a system for managing electronic medical questionnaire information with minimal interaction 10 according to embodiments of the present disclosure, a problem with the prior art will be briefly described with reference to FIG. 1.

Referring to FIG. 1, in the prior art, a patient needs to visit a hospital, fill out answers on a medical questionnaire card provided by the hospital, and the hospital stores the collected PHR information.

The method may only be supported if the patient visits the hospital offline, and the process of providing the appropriate medical questionnaire card to a patient and storing the completed medical questionnaire card back to the server 100 in the hospital may increases the workload of the hospital.

In addition, a process of obtaining a personal information collection consent from the patient is also added, and although the collected information is very important for the patient and the hospital, it is stored only on the server 100 within the hospital, so the utilization of the information is significantly reduced when the patient visits another hospital.

As described above, the conventional method of PHR information collection has been the best method in the past, but currently has various problems, and the inventor of the present disclosure intends to solve these problems through the system for managing electronic medical questionnaire information with minimal interaction 10 according to embodiments of the present disclosure.

Hereinafter, embodiments of the present disclosure will be described in detail with reference to the accompanying drawings.

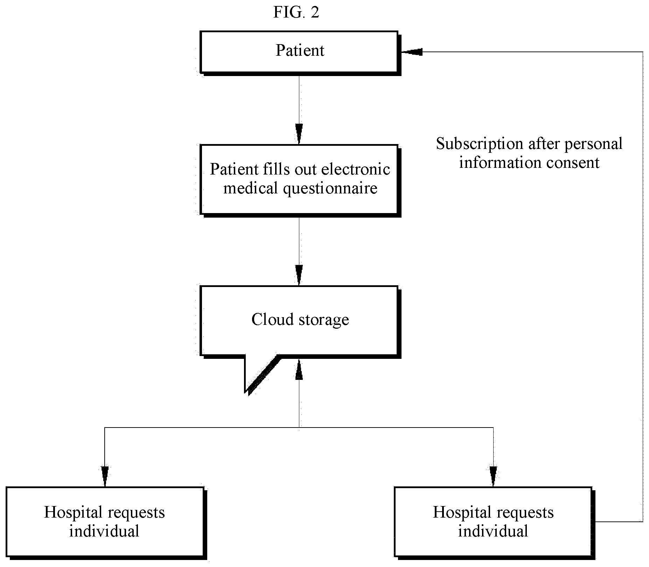

FIG. 2 is a diagram illustrating an example of a method for managing electronic medical questionnaire information with minimal interaction according to an embodiment of the present disclosure.

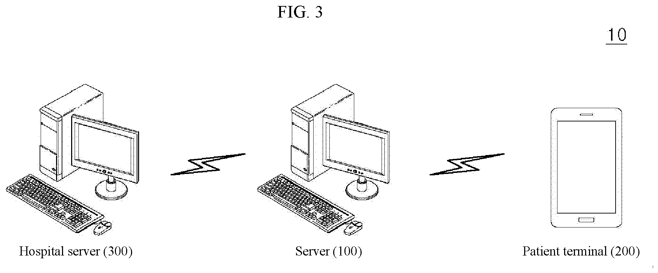

FIG. 3 is a schematic diagram of the system for managing electronic medical questionnaire information with minimal interaction 10 according to an embodiment of the present disclosure.

Referring to FIGS. 2 and 3, the server 100 providing an electronic medical questionnaire information management service with minimal interaction according to an embodiment of the present disclosure may communicate with a patient terminal 200 and a hospital server 300 via a communication device 120.

In this case, a hospital may be a hospital that is subscribed/affiliated to the electronic medical questionnaire information management service provided by the server 100. When a patient visits the hospital off-line or online, the patient may provide personal identifiable information to the hospital. When the hospital provides the personal identifiable information to the server 100 and requests an electronic medical questionnaire, and the patient may automatically subscribe to the service according to the response to the electronic medical questionnaire.

As shown in FIG. 2, ① when a patient registers for a medical appointment at a hospital either offline or online, (2) the hospital requests an electronic medical questionnaire and the personal information collection consent for the patient from the server 100, (3) the server 100 prompts the patient to complete an appropriate electronic medical questionnaire along with obtaining a personal information collection consent, ④ the server 100 receives the results of the patient's response to the electronic medical questionnaire, and (5) the server 100 forwards the results to the hospital.

The system 10 for managing electronic medical questionnaire information with minimal interaction according to an embodiment of the present disclosure will be described in more detail below with reference to other drawings.

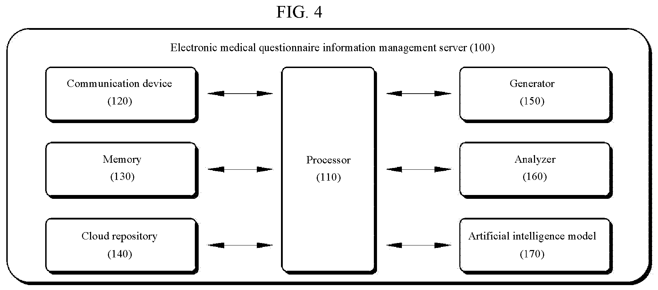

FIG. 4 is a block diagram of the server for managing electronic medical questionnaire information with minimal interaction 100 according to an embodiment of the present disclosure.

Referring to FIG. 4, the server for managing electronic medical questionnaire information with minimal interaction 100 according to an embodiment of the present disclosure may include a processor 110, the communication device 120, a memory 130, a cloud repository 140, a generator 150, an analyzer 160, and an artificial intelligence model 170.

In some embodiments, the server 100 may include fewer or more components than the components illustrated in FIG. 4.

The communication device 120 may communicate with the server 300 of a hospital affiliated/subscribed to an electronic medical questionnaire information management service, and perform communication by providing a link to the patient terminal 200 to which an electronic medical questionnaire is requested from the hospital.

The communication device 120 may include one or more components that enable communication with external devices, and may include at least one of, for example, a broadcast reception module, a wired communication module, a wireless communication module, a short-range communication module, and a location information module.

The wired communication module may include a variety of wired communication modules, such as a Local Area Network (LAN) module, a Wide Area Network (WAN) module, or a Value Added Network (VAN) module, as well as a variety of cable communication modules, such as Universal Serial Bus (USB), High Definition Multimedia Interface (HDMI), Digital Visual Interface (DVI), RS-232 (recommended standard232), power line communication, or plain old telephone service (POTS).

The wireless communication module may include not only a WiFi module and a wireless broadband module, but also wireless communication modules supporting various wireless communication schemes such as global system for mobile communication (GSM), code division multiple access (CDMA), wideband code division multiple access (WCDMA), universal mobile telecommunications system (UMTS), time division multiple access (TDMA), long term evolution (LTE), 4G, 5G, and 6G.

The wireless communication module may include a wireless communication interface including an antenna and a transmitter for transmitting signals. The wireless communication module may further include a signal conversion module that, under control of a controller, modulates a digital control signal output from the controller via the wireless communication interface into an analog radio signal.

The memory 130 may store data supporting various functions of the server for managing electronic medical questionnaire information 100 and programs for operation of the controller, may store input/output data (e.g., music files, still images, moving images, or the like) and may store a number of applications (application programs or applications) running on the a server for managing electronic medical questionnaire information 100, and data and instructions for operation of the server for managing electronic medical questionnaire information 100. At least some of these application programs may be downloaded from an external server 100 through wireless communication.

The memory 130 may include at least one type of storage medium among a flash memory 130 type, a hard disk type, a solid state disk type, an Silicon Disk Drive type (SDD) type, a multimedia card micro type, a card type memory (e.g., SD or XD memory), random access memory (RAM), static random access memory (SRAM), read-only memory (ROM), electrically erasable programmable read-only memory (EEPROM) programmable read-only memory (PROM), magnetic memory 100, magnetic disk, and optical disk. Furthermore, the memory 130 may be a database that is separate from the host device but connected thereto in a wired or wireless manner.

The processor 110 may be implemented with the memory 130 that stores data for an algorithm or program reproducing the algorithm for controlling the operations of components within the server for managing electronic medical questionnaire information 100, and at least one processor 110 that performs the aforementioned operations using the data stored in the memory 130. In this case, the memory 130 and the processor 110 may be implemented as separate chips. Alternatively, the memory 130 and the processor 110 may be implemented as a single chip.

In addition, the processor 110 may control one or more of the aforementioned components in combination to implement various embodiments according to the present disclosure to be described in the drawings below on the device.

In an embodiment of the present disclosure, the server for managing electronic medical questionnaire information 100 may be configured as a cloud server 100, and in some embodiments, may have a cloud repository space separate from the server 100.

In an embodiment of the present disclosure, the server for managing electronic medical questionnaire information 100 may store configurations (e.g., algorithms, instructions, artificial intelligence models 170) for the operation of the processor 110 in the memory 130, and may store data for the electronic medical questionnaire information management service (e.g., electronic medical questionnaire responses, patient personal identification information, etc.) in the cloud repository 140.

The generator 150 may generate various configurations for the service provided by the server 100 in the embodiment of the present disclosure.

More specifically, the generator 150 may generate a link that includes a personal information collection consent and an electronic medical questionnaire to be provided to the patient, and create a patient's account.

In addition, the generator 150 may generate a storage space/location corresponding to the patient's account in the cloud repository 140.

The analyzer 160 may analyze the cumulative medical questionnaire responses collected from the patient to generate useful information for the patient and the hospital.

The analyzer 160 may input the cumulative medical questionnaire responses for the patient into the artificial intelligence model 170, and the artificial intelligence model 170 may output the analysis results for the patient using a trained model.

The details for operations of the generator 150 and the analyzer 160 will be described later.

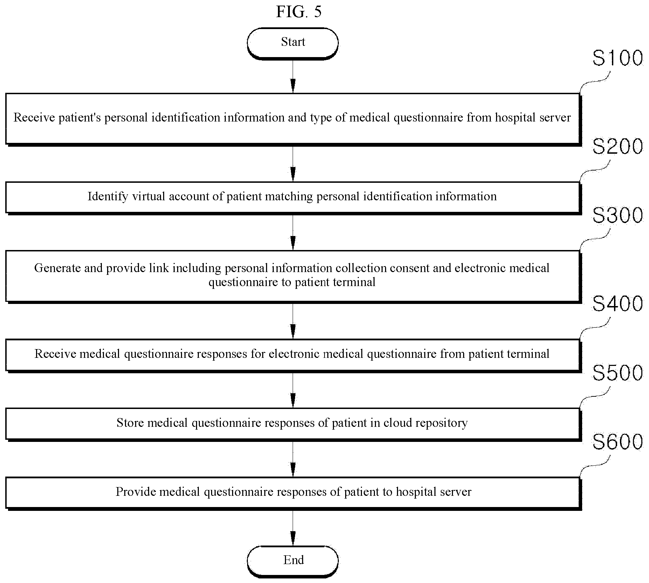

FIG. 5 is a flowchart of a method for managing electronic medical questionnaire information with minimal interaction according to an embodiment of the present disclosure.

Referring to FIG. 5, the method for managing electronic medical questionnaire information with minimal interaction according to an embodiment of the present disclosure may be performed according to the following processes, and each process may be performed differently depending on whether a patient's account exists or does not exist.

The server 100 may receive the patient's personal identification information and the type of a medical questionnaire from a hospital server. (S100)

The processor 110 may identify an account of a patient matching the personal identification information. (S200)

The processor 110 may generate a link including at least one of a personal information collection consent and an electronic medical questionnaire and provide the link to the patient terminal 200. (S300)

The server 100 may receive at least one of the personal information collection consent and the medical questionnaire responses from the patient terminal 200. (S400)

The processor 110 may store the medical questionnaire responses of the patient in the cloud repository 140 corresponding to the patient's account. (S500)

The processor 110 may provide the medical questionnaire responses of patient to the hospital server. (S600)

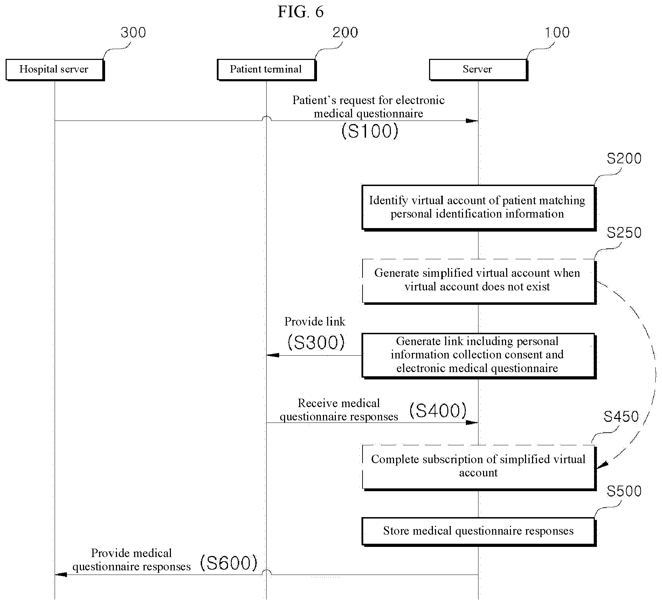

FIG. 6 is a flowchart illustrating a case in FIG. 5 where the patient's account does not exist.

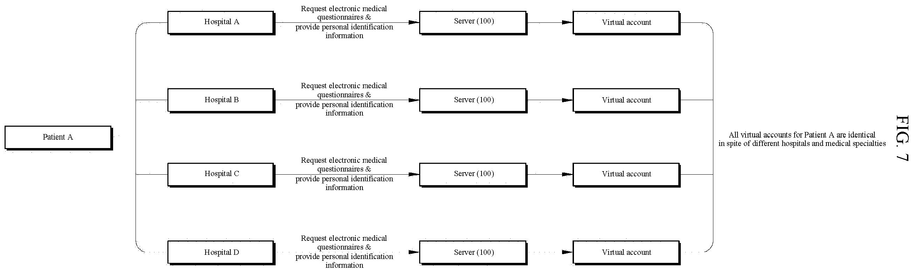

FIG. 7 is a flowchart illustrating a case in FIG. 5 where the patient's account exists.

First, referring to FIG. 6, a process of the case where the patient's account does not exist will be described.

The server 100 may receive a data packet from the hospital server 300 via the communication device 120 that includes personal identification information of a patient and the type of a medical questionnaire to be provided to the patient. (S100)

In this case, the personal identification information of the patient may include the patient's name or telephone number, and any other information capable of identifying the patient may be used as the personal identification information of the patient.

For example, biometric information collected for the patient from the patient terminal 200 may be used to enhance security.

The processor 110 may identify a patient account matching the personal identification information of the patient. (S200)

In this case, when no account is identified that matches the patient's personal identification information, the processor 110 may recognize the patient as a first-time user and perform the process shown in FIG. 6.

When the processor 110 determines that the patient's account does not exist at S200, the processor 110 may generate and provide a link that includes a personal information collection consent and an electronic medical questionnaire to the patient terminal 200. (S300)

Based on the personal information collection consent and medical questionnaire responses by the patient terminal 200, the processor 110 may receive the personal information collection consent and medical questionnaire responses from the patient terminal 200. (S400)

Based on the information, the processor 110 may create an account for the patient and complete the subscription to the electronic medical questionnaire information management service. (S450)

The processor 110 may store the medical questionnaire responses received at S400 in the cloud repository 140 corresponding to the patient's account created at S450. (S500)

In an embodiment, the processor 110 may generate a first code using the patient's full name, generate a second code using the patient's phone number, and combine the first code and second code to create the account. In this case, the code may include at least one of a lowercase/uppercase letter, a number, and a symbol.

In a further embodiment, the processor 110 may generate a first code using the patient's full name, generate a second code using the patient's phone number, generate a third code using the account creation time, and combine the first code, the second code, and the third code to create the account for the patient.

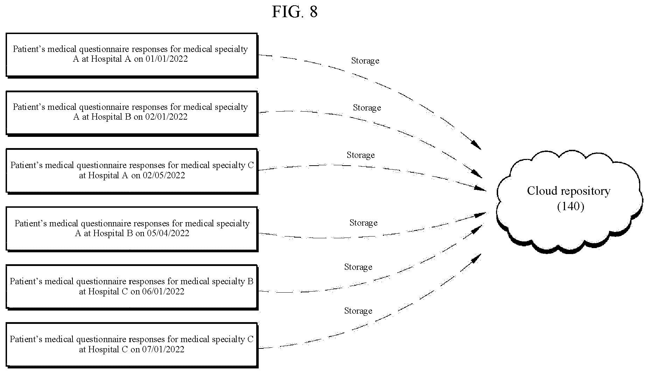

FIG. 8 illustrates an example in which an account is created/verified for the same server 100 even when the same patient visits various hospitals.

In the embodiment of the present disclosure, the processor 110 may create an account for a patient based on the patient's personal identification information, as described above, so that the same account may be used regardless of the hospital or medical specialty that the patient visits, as shown in FIG. 8.

The processor 110 may generate a link including the personal information collection consent and the electronic medical questionnaire, which is to be provided to the patient, by controlling the generator 150.

The server for managing electronic medical questionnaire information 100 according to an embodiment of the present disclosure provides a link including both the personal information collection consent and the electronic medical questionnaire to reduce processes to a single process as compared to the prior art, making it possible to perform the personal information collection consent and the electronic medical questionnaire simultaneously.

When the link is generated, the processor 110 may provide the generated link to the patient terminal 200 via the communication device 120, the generated link being provided via a text message, an MMS message, an instant messaging message, or the like.

In an embodiment, the generator 150 may generate a link address based on the first code and the second code.

In another embodiment, the generator 150 may generate a link address based on the first code, the second code, and the third code.

As an example, the generator 150 may generate a fourth code based on when the link is generated or the medical specialty of the hospital where the patient is being treated. Further, the generator 150 may generate a link address by combining the first code, the second code, and the fourth code. Alternatively, the generator 150 may generate a link address by combining the first code, the second code, the third code, and the fourth code.

In addition, the electronic medical questionnaire information management system 10 according to an embodiment of the present disclosure may simplify or eliminate the procedure from the patient's point of view as compared to the prior art, since a patient is automatically subscribed to the service when he or she goes to a hospital for an online or offline consultation, consents to the collection of personal information, and completes an electronic medical questionnaire without having to go through a separate subscription procedure.

In some embodiments, if the patient does not have an account and has no history of subscribing to the service, the generator 150 may provide information at the top of the link informing the patient that he or she will be automatically subscribed to the service if he or she consents to the collection of personal information and completes an electronic medical questionnaire.

In an embodiment of the present disclosure, the link provided by the server 100 may be a web link, and a web page may be automatically connected when the patient receives the link on the terminal.

The link may include a web page that asks the patient to consent to the collection of personal information and a web page for an electronic medical questionnaire corresponding to the hospital and medical specialty visited by the patient, allowing the patient to consent to the collection of personal information and immediately respond to the electronic medical questionnaire.

For example, the link may include a message that asks the patient to proceed to the electronic medical questionnaire at the bottom if the patient consents to the collection of personal information, and the patient's personal information collection consent may be automatically processed when the patient responds to the electronic medical questionnaire. As described above, the personal information collection consent and the electronic medical questionnaire included within the link may be processed in various ways.

In some embodiments, the processor 110 may create a simplified account for the patient based on the personal identification information if the patient's account does not exist, and subscription may be completed for the simplified account upon receipt of the responses of the electronic medical questionnaire from the patient terminal.

Next, a process when a patient's account exists will be described with reference to FIG. 7.

When the processor 110 determines that the patient's account exists at S200, the processor 110 may generate and provide a link including an electronic medical questionnaire to the patient terminal 200. (S300)

The processor 110 may receive the medical questionnaire responses from the patient terminal 200 when inputs for the electronic medical questionnaire are received from the patient terminal 200. (S400)

The processor 110 may store the medical questionnaire responses received at S400 in the cloud repository 140 corresponding to the patient's account. (S500)

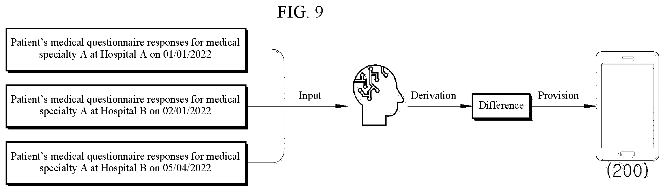

FIG. 9 illustrates an example in which various medical questionnaire responses of the same patient are stored in the same account in the cloud repository 140 according to FIG. 8.

In an embodiment of the present disclosure, the processor 110 may generate and allocate a storage space in the cloud repository 140 according to the patient's account, and in this case, allocating a storage space may mean creating a folder for that patient within the cloud repository 140.

However, this is only an illustrative example to aid understanding of storing the medical questionnaire responses in a storage space corresponding to the patient's account in the cloud repository 140, and the present disclosure is not intended to be limited thereto.

The processor 110 may store the medical questionnaire responses received from the patient terminal 200 for the electronic medical questionnaire in the cloud repository 140 in a cumulative manner after the patient's subscription to the service is completed, and when a request for provision of the cumulative medical questionnaire responses for the patient is received from a specified hospital server 300, the processor 110 may extract the medical questionnaire responses for a period corresponding to the request for provision and provide them to the hospital server 300.

In this case, the request for provision of the cumulative medical questionnaire responses for the patient received from the hospital server 300 may include a medical specialty and a medical period, and the processor 110 may extract the medical questionnaire responses corresponding to the requested medical specialty and medical period from among the medical questionnaire responses accumulated for the patient, generate a data packet, and provide the data packet to the corresponding hospital server 300. (e.g., medical specialty: gastroenterology and medical period: 2020-2021)

In an embodiment of the present disclosure, the processor 110 may categorize the medical questionnaire responses according to the patient's medical specialty and store the medical questionnaire responses in a cumulative manner.

In the embodiment described above, the generator 150 may generate a fourth code based on the medical specialty of the hospital where the patient is being treated.

The processor 110 may determine a storage space/location in the cloud repository 140 based on the medical specialty of the hospital where the patient is being treated or the fourth code included in a link provided to the patient terminal 200, and store the medical questionnaire responses received from the patient terminal 200 in the determined storage space/location in a cumulative manner.

Thus, the medical questionnaire responses for the same medical specialty of the same patient may be stored in the same storage space/location in the cloud repository 140.

In addition, as described above, when a link is generated based on at least one of the first code, the second code, the third code, and the fourth code and provided to the patient terminal 200, the link includes information on the patient's account and the medical specialty, thus making it easy to check and store the information as well as to generate the link.

In an embodiment, the processor 110 may control the analyzer 160 to analyze the patient's medical questionnaire responses and provide useful information to the patient and the hospital.

More specifically, the processor 110 may store the electronic medical questionnaire responses received from the patient terminal 200 in a cumulative manner after the patient has subscribed to the service, and when the medical questionnaire responses for the new electronic medical questionnaire (hereinafter, 'new medical questionnaire responses') are received from the corresponding patient terminal 200, the processor 110 may compare the new medical questionnaire responses with the cumulative medical questionnaire responses to derive differences therebetween, and provide the derived differences to the patient terminal 200.

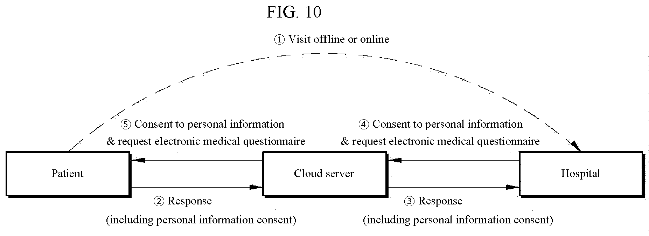

FIG. 10 is a diagram illustrating an example in which the artificial intelligence model 170 compares the patient's medical questionnaire responses at different times to derive differences and providing the differences to the patient.

In an embodiment of the present disclosure, the artificial intelligence model 170 is a trained model, and a method of deriving various medical questionnaire responses and recommended information according to the patient's medical questionnaire responses has been trained.

Therefore, the artificial intelligence model 170 may analyze the patient's cumulative medical questionnaire responses to derive and provide the patient with necessary recommended information, and in this case, as the recommended information, exercise, eating habits, nutritional supplements, treatment, or the like may be applied.

In addition, the artificial intelligence model 170 may generate health information related to the patient by analyzing the patient's cumulative medical questionnaire responses, and when new medical questionnaire responses are received for the patient, compare the generated health information with the new medical questionnaire responses to derive differences.

For example, the artificial intelligence model 170 may derive improvement points, deterioration points, warning points, or the like as differences such as the patient's inability to properly manage his health, the patient's aspects of not properly managing his health compared to before, and the patient's aspects of properly managing his health compared to before.

For example, when the amount of alcohol consumed has decreased compared to before in a situation where the patient's liver health is not good, the processor 110 may derive improvement points as differences and provide the improvement points to the patient terminal 200.

As another example, when the amount of alcohol consumed has not decreased or increased more compared to before in a situation where the patient's liver health is not good, the processor 110 may derive deterioration points as differences and not only provide the deterioration points to the patient terminal 200, but also provide a warning message depending on the degree of difference.

In addition, the processor 110 may provide recommended information on nutritional supplements, health foods, and exercise required for the patient based on the derived differences.

The method according to an embodiment of the present disclosure described above may be implemented as a program (or application) to be executed in combination with the server 100, which is hardware, and stored in a medium.

The above-described program may include codes coded in a computer language, such as C, C++, JAVA, or a machine language, which are readable by a processor (CPU) of the computer through a device interface of the computer such that the computer reads the program and executes the methods implemented by the program. The codes may include functional codes associated with a function defining functions necessary to execute the methods or the like, and include control codes associated with an execution procedure necessary for the processor 110 of the computer to execute the functions according to a predetermined procedure. In addition, the codes may further include memory 130 reference codes indicating at which location (address number) of the computer's internal or external memory 130, additional information or media required for the computer's processor 110 to execute the functions can be referenced. In addition, when the processor 110 of the computer needs to communicate with any other computer or the server 100 located remotely to execute the above functions, codes may further include communication-related codes for how to communicate with any other remote computer or the server 100 using a communication module of the computer, and what information or media to transmit/receive during communication.

The stored medium refers to a medium that stores data semi-permanently rather than a medium storing data for a very short time, such as a register, a cache, and the memory 130, and is readable by an apparatus. Specifically, examples of the storage medium may include, but are not limited to, a ROM, a RAM, a CD-ROM, a magnetic tape, a floppy disk, an optical data storage device, and the like. That is, the program may be stored in various recording media on various servers 100 to which the computer can access or various recording media on the computer of a user. The medium may also be distributed to a computer system 10 connected thereto through a network and store computer readable codes in a distributed manner.

The steps of a method or algorithm described in connection with the embodiments of the present disclosure may be implemented directly in hardware, in a software module executed by hardware, or in a combination thereof. The software module may reside in a random access memory (RAM), a read only memory (ROM), an erasable programmable ROM (EPROM), an electrically erasable programmable ROM (EEPROM), a flash memory, a hard disk, a removable disk, a CD-ROM, or in a computer readable recording medium that is well known in the art.

Although embodiments of the present disclosure have been described above with reference to the accompanying drawings, it is understood that those skilled in the art to which the present disclosure pertains may implement the present disclosure in other specific forms without changing the technical spirit or essential features thereof. Therefore, it should be understood that the embodiments described above are illustrative in all respects and not restrictive.

## Claims

1. A server for managing electronic medical questionnaire information with minimal interaction comprising: a communication device configured to receive, from a hospital server, a data packet including personal identification information of a patient and a type of a medical questionnaire to be provided to the patient; and
a processor;
wherein the processor is configured to:
identify an account of the patient matching the personal identification information,
generate a link including at least one of a personal information collection consent and an electronic medical questionnaire, the link being to be provided to the patient;
provide the generated link to a terminal of the patient, and receive, from the terminal of the patient, medical questionnaire responses for the electronic medical questionnaire;
store the received medical questionnaire responses in a cloud repository corresponding to the account of the patient; and
provide the received medical questionnaire response to the hospital server via the communication device.

2. The server of claim 1, wherein the processor is configured to:
create a simplified account for the patient based on the personal identification information when the account of the patient does not exist in the server upon identification of the account; and
complete subscription for the simplified account when receiving the medical questionnaire responses for the electronic medical questionnaire from the terminal of the patient,
wherein the personal identification information includes a full name and a phone number of the patient.

3. The server of claim 2, wherein the processor is configured to:
store the medical questionnaire responses received from the terminal of the patient for the electronic medical questionnaire in a cumulative manner after the subscription is completed; and
when a request for provision of the cumulative medical questionnaire responses for the patient is received from a specified hospital server, provide the medical questionnaire responses for a period corresponding to the request for provision to the specified hospital server.

4. The server of claim 4, wherein the processor is configured to:
categorize the medical questionnaire responses according to a medical specialty of the patient; and
store the medical questionnaire responses in a cumulative manner.

5. The server of claim 2, wherein the processor is configured to:
store the medical questionnaire responses received from the terminal of the patient for the electronic medical questionnaire in a cumulative manner after the subscription is completed; and
when medical questionnaire responses for a new electronic medical questionnaire are received from the terminal of the patient, compare the new medical questionnaire responses with the cumulative medical questionnaire responses to derive differences therebetween; and
provide the derived differences to the terminal of the patient.

6. The server of claim 2, wherein the processor is configured to allow the patient to use the subscribed account identically even when the patient uses a hospital other than the hospital.

7. The server of claim 6, wherein the server is affiliated with a non-medical institution.

8. A method for managing electronic medical questionnaire information with minimal interaction, the method being performed by a server, comprising:
receiving, from a hospital server, a data packet including personal identification information of a patient and a type of a medical questionnaire to be provided to the patient;
identifying an account of the patient matching the personal identification information;
generating a link including at least one of a personal information collection consent and an electronic medical questionnaire, and providing the generated link to a terminal of the patient;
receiving, from the terminal of the patient, medical questionnaire responses for the electronic medical questionnaire;
storing the received medical questionnaire responses in a cloud repository corresponding to the account of the patient; and
providing the received medical questionnaire response to the hospital server.

9. The method of claim 8, wherein the identifying of the account further includes creating a simplified account for the patient based on the personal identification information when the account of the patient does not exist in the server upon identification of the account; and
wherein the receiving of the medical questionnaire responses further includes completing subscription for the simplified account when receiving the medical questionnaire responses for the electronic medical questionnaire from the terminal of the patient,
wherein the personal identification information includes a full name and a phone number of the patient.

10. The method of claim 9, wherein the server is configured to:
store the medical questionnaire responses received from the terminal of the patient for the electronic medical questionnaire in a cumulative manner after the subscription is completed;
when a request for provision of the cumulative medical questionnaire responses for the patient is received from a specified hospital server; and
provide the medical questionnaire responses for a period corresponding to the request for provision to the specified hospital server.

11. The method of claim 10, wherein the server is configured to:
categorize the medical questionnaire responses according to a medical specialty of the patient; and
store the medical questionnaire responses in a cumulative manner.

12. The method of claim 9, wherein the server is configured to:
store the medical questionnaire responses received from the terminal of the patient for the electronic medical questionnaire in a cumulative manner after the subscription is completed;
when medical questionnaire responses (hereinafter, referred to as new medical questionnaire responses') for a new electronic medical questionnaire are received from the terminal of the patient;
compare the new medical questionnaire responses with the cumulative medical questionnaire responses to derive differences therebetween; and
provide the derived differences to the terminal of the patient.

13. The method of claim 9, wherein the server is configured to allow the patient to use the subscribed account identically even when the patient uses a hospital other than the hospital.

14. The method of claim 13, wherein the server is affiliated with a non-medical institution.

15. A computer-readable recording medium, which is combined with a computer as hardware and stores an electronic medical questionnaire information management program for executing the method for managing electronic medical questionnaire information with minimal interaction of claim 8.
